# EUROPEAN PATENT APPLICATION

(11) **EP 0 919 533 A1**
(43) Date of publication of application: **02.06.1999**
(21) Application number: 98402852.2
(22) Date of filing: 17.11.1998
(51) Int. Cl.: C07C 46/04, C07C 50/12, C07C 205/46, C07C 45/28, C25B 1/00

(54) **Electrosynthesis of aromatic quinones**

(30) Priority: 25.11.1997 US 978518
(71) Applicant: HYDRO-QUEBEC, Montréal Québec H2Z 1A4 (CA)
(72) Inventor: Harrison, Stephen, Shawinigan G9N 1B3 (Québec) (CA); Fiset, Ghislain, Cap-de-la-Madeleine G8T 5L8 (Québec) (CA); Mahdavi, Behzad, Shawinigan G9N 7P3 (Québec) (CA)
(74) Representative: Bertrand, Didier

(57) **Abstract**

Oxidation of alkyl and aromatic compounds using ceric ions. The alkyl and aromatic compounds are in solution in a C₅ to C₁₅ alkane, preferably hexane or n-heptane or n-decane or cyclohexane, at a concentration preferably between 0.1 to 3.0 molar, such that the carbonyl compound obtained is at a concentration which is above its minimum solubility in the alkanes and below the maximum solubility of the aromatic or alkyl aromatic compound in the alkanes. The product obtained is purer, i.e. there are lesser by-products associated therewith, and chemical yields are substantially improved.

## Description

### BACKGROUND OF INVENTION

### (a) Field of the Invention

The invention relates to a process for oxidizing alkyl and aromatic compounds using ceric ions. More specifically, the invention relates to the indirect electrosynthesis of carbonyl containing compounds using ceric ions for the oxidation of alkyl and aromatic compounds, the latter being in solution in a C₅ to C₁₅ alkanes and wherein the concentration of reagent in the solvent is critical so as to provide purer carbonyl compounds and less by-products, and substantially improved chemical yields.

### (b) Description of Prior Art

U.S. Patent No. 5,296,107 issued March 22, 1994, Stephen Harrison inventor, describes inter alia a process whereby aromatic and alkyl aromatic reactants are oxidized in an electrochemical cell wherein cerium^{III} is converted to cerium ^{IV}. Now, it has been found that this process which is on the one hand very interesting commercially, has some disadvantages in that the oxidized product is not as pure as would be expected and is most of the time accompanied by a certain amount of by-products. Reference is also made to U.S. Patent no. 5,516,407 issued May 14, 1996, which is a continuation-in-part of U.S. 5,296,107.

Prior art of interest also includes United States Patent No. 3,873,580, British patents GB 1 360 904, 1 203 434 and 1 192 037, all issued to R. A. C. Rennie, and a two-part article authored by D. Pletcher and E. Valdes entitled "Studies of the Ce(III)/Ce(IV) Couple in Multiphase Systems Containing a Phase Transfer Reagent" published in *Electrochemica Acta,* vol. 334, no. 4. pages 499-515 (1987).

Each patent generally teaches the oxidation of aromatic hydrocarbons by an aqueous acidic solution of an oxidizing agent, where the aromatic hydrocarbon is dissolved in an organic solvent. Suitable organic solvents identified are chlorinated hydrocarbons, petroleum solvents and aliphatic, particularly C₅-C₁₅ hydrocarbons, including hexane. Each patent also recognizes that the processes may be batchwise or continuous.

U.S. 3,873,580 and GB 1 360 904 teach the use of hexane as the organic solvent for the dissolution of various aromatic hydrocarbons, including naphthalene (0.1 M), diphenyl (0.1 M), anthracene (0.1 M) and toluene (0.2 M). The oxidizing agent is persulfate anion, which is electrolytically regenerated from sulfate ion in a separate cell. Cerium, when present at about 0.01 to 1 mole/mole organic substrate, appears to be a catalyst to the oxidation. Oxidation preferably occurs in the presence of a co-catalyst such as silver or iron.

With one exception, after the desired time for the oxidation, carbonyl-containing products remained in the liquid phase. In example 4 of U.S. 3,873,580, solid 9, 10-anthraquinone was recovered, after two hours' reaction time, by filtration. The reaction mixture contained anthracene (0.1 M solution in hexane) and ceric ion in the presence of Ag⁺.

GB 1 203 434 teaches oxidation by ceric ion of 2,6-di-t-butylphenol dissolved in hexane to form a 0.05 M organic phase. Yield of 2,2',6,6'-tetrabutyldiphenoquinone was "substantially quantitative". This patent also teaches that after the desired time for the oxidation, carbonyl-containing products were in the liquid phase. Desired products were obtained from the liquid phase by evaporative or crystallization techniques.

GB 1 192 037 teaches the dissolution of diphenyl in hexane to form a 0.19 M solution. Ceric ion was the oxidizing agent. A 70% yield of 2-phenylquinone was obtained. This patent teaches that quinones were obtained from the liquid phase by evaporative or crystallization techniques.

Part 1 of the Pletcher et al. article teaches electrochemical regeneration of ceric ion in an undivided cell in which the electrolyte was an emulsion of an aqueous acidic phase and an organic solvent such as toluene, methylene chloride and hexane. Tributylphosphate or tetrabutylammonium ions were the interphase transfer agents. The second part of the article teaches indirect cerium-mediated oxidation of a 0.03 M solution of anthracene in n-heptane where the aqueous phase contains nitric acid. Yield of anthraquinone was "close to quantitative" when tributylphosphate was the transfer agent and "typically" 60% when tetrabutylammonium was the transfer agent. At the end of the reaction, a product was obtained by evaporation of the organic phase.

None of these references teaches the oxidization of aromatic hydrocarbons by ceric ion where the concentration of aromatic hydrocarbon in the solvent is critical. Also, none of these references teach the precipitation of a carbonyl-containing reaction product from the oxidation of an aromatic hydrocarbon by ceric ion.

It is an object of the present invention to provide a process for the oxidization of alkyl and aromatic compounds which ensures time/efficiency savings.

It is another object of the present invention to provide an oxidization process wherein the degradation of carbonyl-containing reaction products such as naphthoquinone to the corresponding carboxylic acids is minimized.

It is another object of the invention to provide a process of oxidizing alkyl and aromatic compounds where high concentrations of the organic substrate in the organic phase promotes precipitation of the desired carbonyl-containing product, facilitating its separation from the reaction mixture.

It is yet another object of the present invention to provide an oxidizing process which eliminates chlorinated solvent from the process thereby eliminating the potential break down of these solvents into chloride ions which results in corrosion problems with both cathodes and anodes and other ancillary equipment.

### SUMMARY OF THE INVENTION

In accordance with the invention there is provided a process for oxidizing aromatic compounds and alkyl aromatic compounds, comprising:
(a) reacting an aromatic or alkyl aromatic reactant in an organic solvent with ceric ions in an aqueous acidic solution to form cerous ions and a carbonyl-containing product, wherein the organic solvent is selected from the group comprising C₅ to C₁₅ alkanes and wherein the concentration of the aromatic or alkyl aromatic compound in the organic solvent is such that the carbonyl-containing product obtained is at a concentration which is above its minimum solubility in the alkanes and below the maximum solubility of the aromatic or alkyl aromatic compound in the alkanes.
   In accordance with a preferred embodiment of the invention, the process is carried out by oxidizing naphthalene, nitronaphthalene or methyl-naphthalene, and the solvent is preferably straight chain or cyclic hydrocarbons, such as heptane, hexane, octane, decane or cyclohexane. The most preferred solvent include n-hexane, n-heptane, n-octane and n-decane.
   In accordance with another embodiment of the invention, the invention further comprises the step of
(b) separating the solution containing unreacted aromatic or alkyl aromatic compound from the carbonyl-containing compound for recycling to step (a).

Preferably, when the reactant is nitronaphthalene, the solvent is cyclohexane while the concentration of nitronaphthalene in cyclohexane is from about 0.02 to about 1.0 molar, most preferably about 0.4. Alternatively, the reactant can be methyl-naphthane and the solvent is n-decane, with a methyl-naphthane concentration of about 0.05 to 3.0 molar. when the reactant is naphthalene, the solvent is preferably n-heptane, and the concentration of naphthalene is from about 0.02 to about 1.1 molar.

In accordance with yet another embodiment, the invention relates to a process for producing aromatic aldehydes, ketones and quinones, comprising the steps of:
(a) reacting a solution of from about 0.05 to about 3.0 molar of an aromatic or alkyl aromatic compound dissolved in a C₅ to C₁₅ alkane wiith ceric methanesulfonate to produce a product mixture containing an unreacted aromatic or alkyl aromatic compound, cerous ions and a solid carbonyl-containing product;
(b) separating from the product mixture, a solution of unreacted aromatic or alkyl aromatic compound and recycling the unreacted compound to step (a);
(c) separating the cerous ion from the product mixture;
(d) recovering trace organic substances by liquid - liquid extraction of the electrolyte with an organic solvent;
(e) electrolytically regenerating the cerous ion to form the ceric ion and recycling the ceric ion to step (a) for continued processing.

In accordance with yet another embodiment of the invention, the invention relates to a process for producing aromatic quinones, aldehydes or ketones from aromatic or alkyl aromatic compounds, comprising the steps of:
(a) dissolving an aromatic or alkyl aromatic compound in an organic solvent to form a reactant solution, the organic solvent being selected from the group consisting of C₅ to C₁₅ alkanes;
(b) contacting the reactant solution with an aqueous acidic solution of ceric ion to form a product mixture including a liquid aqueous phase, and a liquid organic phase, wherein the concentration of the aromatic or alkyl aromatic compound in the reactant solution is adequate to produce an organic product comprising aromatic quinone, aldehyde or ketone in solid form, and wherein the liquid aqueous phase comprises cerous ion, and the organic phase comprises the organic solvent and unreacted aromatic or alkyl aromatic compounds;
(c) separating the liquid organic phase containing the unreacted aromatic or alkyl aromatic compounds and recycling them to step (b);
(d) separating the liquid aqueous phase including the cerous ion, electrolytically regenerating the cerous ion to ceric ion, and recycling the ceric ion to step (b); and
(e) separating the solid organic product from the product mixture.

### BRIEF DESCRIPTION OF DRAWINGS

The invention is illustrated by but is not restricted to the annexed drawing in which:

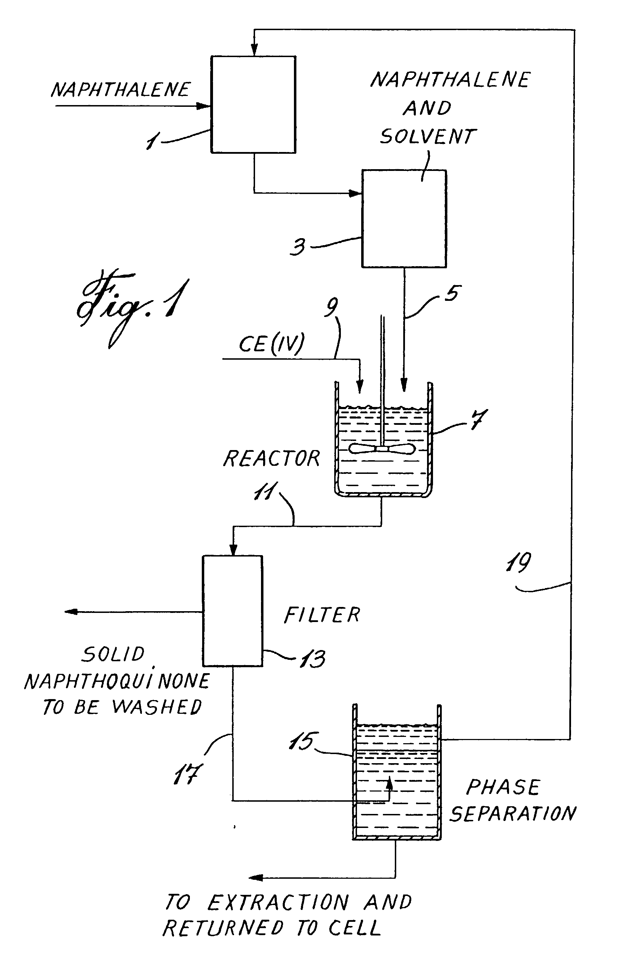

Figure 1 is a flow sheet illustrating a process according to the invention;
Figure 2 shows a modification of the process illustrated in Figure 1.

### DESCRIPTION OF PREFERRED EMBODIMENT

With reference to the drawings it will be seen that naphthalene is supplied to a reservoir 1 which feeds into a container 3 containing an organic solvent to provide a mixture of naphthalene and solvent such as n-heptane. The mixture is fed via duct 5 into reactor 7 which separately receives via duct 9 ceric ions such as in the form of an acidic solution of ceric methanesulphonate. The reaction is allowed to take place and the reaction mixture is fed via duct 11 to a filter 13 which separates naphthoquinone from the solvent and unreacted naphthalene and the solvent are sent to a phase separationunit 15 via duct 17 and the remains are recycled to reservoir 1 via duct 19.

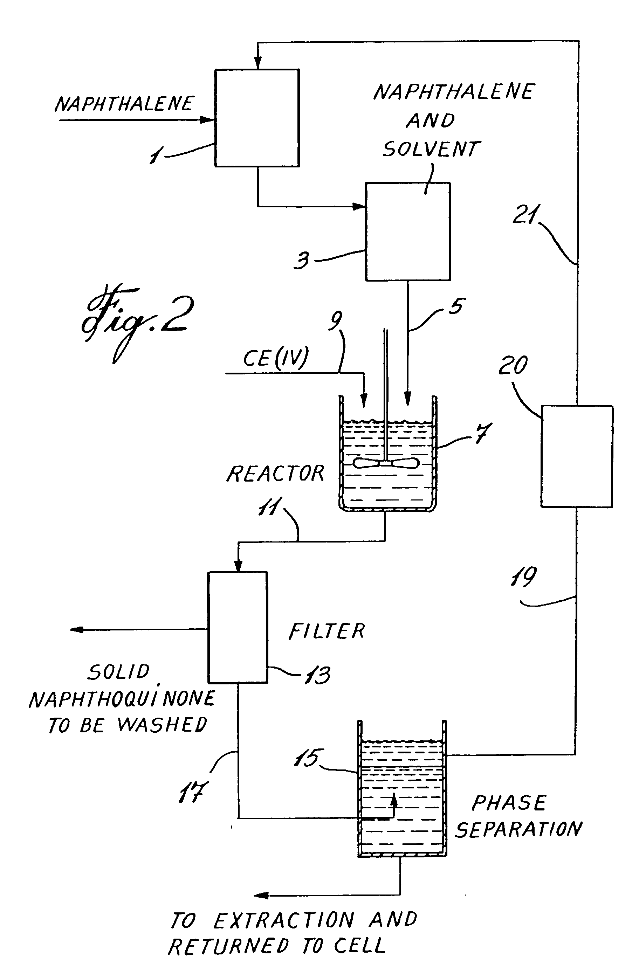

According to Figure 2, the solvent and unreacted naphthalene is sent to a solvent stage 20 before returning to step 7 via line 21. Such processes could include distillation, crystallization, evaporation or other similar technique

The invention is illustrated by means of the following examples

### Example 1

### Stage 1

Naphthalene dissolved in n-heptane (concentrations range is 0.02 to 0.8 M) is reacted with an acidic solution (2.5 to 4.0. M) of ceric methanesulfonate (0.4 to 0.8 M) at 60 °C. The product, naphthoquinone is produced in sufficient concentration (the saturation point of naphthoquinone in such solvents is around 0.02M) that it can be separated as a solid by filtering or by centrifugation from the organic phase while the aqueous phase contains cerous methanesulfonate and methanesulfonic acid. The unreacted naphthalene remains in the n-heptane and can then be recycled back to the naphthalene preparation reservoir after phase separation. The reacting phases are allowed to settle and the cerous solution is returned to an electrochemical cell. The presence of solids in the reactor reduce the reaction time as a continuous process from over 50 minutes to less than 5 minutes. The conversion and yield are greater and the overall process economics measured by energy consumption are lower. The results of the continuous process are shown in Table 1.

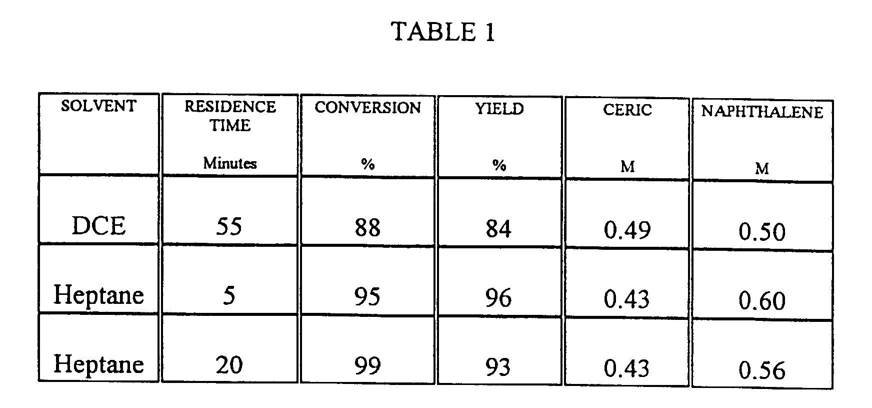

### Stage 2

Cerous methanesulfonate in methanesulfonic acid is fed to the anode compartment of an electrochemical cell where it is oxidized back to ceric methanesulfonate and returned to stage 1.

### Example 2

Molten 1-nitronaphthalene is mixed continuously with cyclohexane at the rate of 2.4 kg/h and 12 kg/h respectively and fed into an agitated vessel. At the same time a ceric solution (0.6 M) in methanesulfonic acid solution is fed at a rate of 300 litres per hour into the same reactor vessel. The mean residence time being 6 minutes. At the exit of the vessel the product nitronaphthoquinone is separated from the two liquid phases by filtration. The liquid phases are fed into a decanter where the lighter cyclohexane phase containing any unreacted nitronaphthalene is siphoned off and returned to the solution make-up for further mixing with fresh nitronaphthalene.

The heavier acid phase is returned to the cell after electrolyte purification. The conversion of nitronaphthalene is 90% and the chemical yield is 85%.

### Example 3

200 parts of an aqueous solution containing ceric methanesulfonate (0.4 M) in methanesulfonic acid (3M) in water was charged in a glass vessel equipped with an agitator. The solution was stirred vigorously and heated to 60°C. Then, 1.14 parts of naphthalene in 15 parts of iso-octane was added to the solution and mixed for 3 minutes. After completion of the reaction, the mixing was stopped and the reaction mixture was transferred to a separation funnel. The reaction mixture was extracted with 2 x 40 parts of dichloroethane. Quantitative analysis by gas chromatography gave a yield of 72% for naphthoquinone.

### Example 4

200 parts of an aqueous solution containing ceric methanesulfonate (0.4M) in methanesulfonic acid (3M) in water was charged in a glass vessel equipped with a agitation. The solution was stirred vigorously and heated to 60°C. Then, 1.14 parts of naphthalene in 15 parts of hexane was added to the solution and mixed for 3 minutes. After completion of the reaction, the mixing was stopped and the reaction mixture was transferred to a separation funnel. The reaction mixture was extracted with 2 x 40 parts of dichloroethane. Quantitative analysis by gas chromatography gave a yield of 80% for naphthoquinone.

### Example 5

200 parts of an aqueous solution containing ceric methanesulfonate (0.4 M) in methanesulfonic acid (3M) in water was charged in a glass vessel equipped with an agitator. The solution was stirred vigorously and heated to 60° C. Then, 1.14 parts of naphthalene in 15 parts of cyclohexane was added to the solution and mixed for 3 minutes. After completion of the reaction, the mixing was stopped and the reaction mixture was transferred to a separation funnel. The reaction mixture was extracted with 2 x 40 parts of dichloroethane. Quantitative analysis by gas chromatography gave a yield of 81% for naphthoquinone.

### Example 6

200 parts of an aqueous solution containing ceric methanesulfonate (0.4 M) in methane sulfonic acid (3M) in water was charged in a glass vessel equipped with a agitation. The solution was stirred vigorously and heated to 60°C. Then, 1.14 parts of naphthalene in 15 parts of n-heptane was added to the solution and mixed for 3 minutes. After completion of the reaction, the mixing was stopped and the reaction was transferred to a separation funnel. The reaction mixture was extracted with 2 x 40 parts of dichloroethane. Quantitative analysis by gas chromatography gave a yield of 75 % for naphthoquinone.

### Example 7

200 parts of an aqueous solution containing ceric methanesulfonate (0.4 M) in methanesulfonic acid (3M) in water was charged in a glass vessel equipped with a agitation. The solution was stirred vigorously and heated to 60°C. Then, 1.14 parts of naphthalene in 15 parts of pentane was added to the solution and mixed for 3 minutes. After completion of the reaction, the mixing was stopped and the reaction mixture was transferred to a separation funnel. The reaction mixture was extracted with 2 x 40 parts of dichloroethane. Quantitative analysis by gas chromatography gave a yield of 53% for naphthoquinone.

### Example 8

200 parts of an aqueous solution containing ceric methanesulfonate (0.04 M) in methanesulfonic acid (3M) in water was charged in a glass vessel equipped with a agitation. The solution was stirred vigorously and heated to 60°C. Then, 1.14 parts of naphthalene in 15 parts of cyclopentane was added to the solution and mixed for 3 minutes. After completion of the reaction, the mixing was stopped and the reaction mixture was transferred to a separation funnel. The reaction mixture was extracted with 2 x 40 parts of dichloroethane. Quantitative analysis by gas chromatography gave a yield of 50% for naphthoquinone.

### Example 9

Procedure for the synthesis of vitamin K3 (menadione)

To a 200 mL solution of cerium in methanesulfonic acid (Ce(Tot)=lM; Ce(VI)-0.25 M; methanesulfonic acid =2.3 M; Cr(VI) = 0.59 g (3%) in the form of CrO₃). to which is added of 0.97 g of 2-methyl-naphthalene dissolved in 10 mL of n-decane. The mixture is stirred at 9000 r.p.m. during 15 minutes. The menadione obtained is a solid product and recovery of the menadione is facilitated. The cerium solution is extracted once the menadione is removed with 50 mL of cyclohexane to recover the dissolved menadione from the acid phase. The yield of the reaction is 70% with a conversion higher than 95%.

Other tests were made to determine the effects of changing the nature of the solvents in a batch process. Reference is made to Table II.

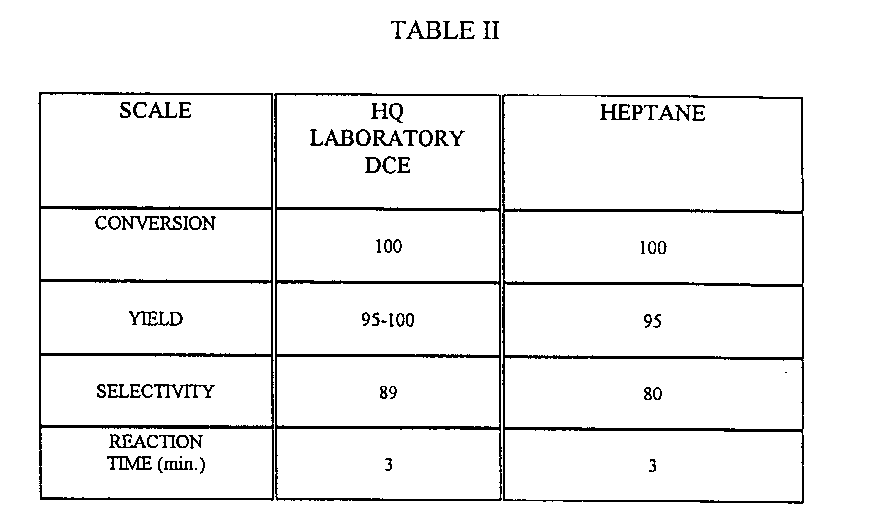

As a batch process the results are comparable with DCE and heptane as solvents. It is therefore unexpected that the reaction time should be that much shorter when operating as a continuous process. The prior art is loaded with references using heptane and hexane and similar solvents for these reactions. They, however, use very low concentrations of naphthalene in the solvent in order to prevent precipitation of the product.

Until now it has been essential to completely convert naphthalene to naphthoquinone because of the difficulty and expense of zone refining naphthalene for recycling back to the oxidation reactor. Complete conversion of naphthalene results in a process penalty due to over oxidation of naphthalene to phthalic acid (i.e. lower yield and a waste disposal problem). The rate of production of phthalic acid in the process according to the invention is around 0.2% up to a maximum of 0.5%.

According to the process of the invention, it is not essential to completely convert naphthalene to naphthoquinone so lower reaction times are possible.

In the present invention the concentration is much greater than those used by Pletcher and Rennie. The fact that applicant uses higher concentrations result in a different method of product recovery.

## Claims

1. A process for oxidizing aromatic compounds and alkyl aromatic compounds, comprising:
(a) reacting an aromatic or alkyl aromatic reactant in an organic solvent with ceric ions in an aqueous acidic solution to form cerous ions and a carbonyl-containing product, wherein the organic solvent is selected from the group comprising C₅ to C₁₅ alkanes and wherein the concentration of the aromatic or alkyl aromatic reactant in the organic solvent is such that the carbonyl-containing product obtained is at a concentration above its saturation point in said alkanes such that it will precipitate and below the maximum solubility of said aromatic or alkyl aromatic reactant in said alkanes, thereby resulting in simultaneous reaction and production of solid product.

2. A process according to claim 1, which comprises oxidizing naphthalene.

3. A process according to claim 1, which comprises oxidizing nitronaphthalene.

4. A process according to claim 1, which comprises oxidizing methyl-naphthalene.

5. A process according to claim 1, wherein said alkanes are selected from the group consisting of heptane, hexane and cyclohexane.

6. A process according to claim 1, wherein said alkanes are straight chain alkanes.

7. A process according to claim 6, wherein said straight chain alkanes are selected from the group comprising n-hexane, n-heptane and n-octane, n-decane.

8. A process according to claim 1, wherein said reactant comprises nitronaphthalene and said organic solvent comprises cyclohexane.

9. A process according to claim 1, wherein said concentration of nitronaphthalene in cyclohexane is about 0.05 to 1.0 molar.

10. A process acording to claim 1, wherein said reactant comprises methyl-naphthalene and said organic solvent comprises n-decane.

11. A process according to claim 10, wherein said concentration of methyl-naphthalene is about 0.05 to 3.0 molar.

12. A process according to claim 1, further comprising the step of
(b) separating the solution containing unreacted aromatic or alkyl aromatic compound from said carbonyl-containing compound for recycling to step (a).

13. The process according to claim 2, wherein said alkane is n-heptane, said concentration of naphthalene in n-heptane is from about 0.02 to about 1.1 molar.

14. Process for producing aromatic aldehydes, ketones and quinones, comprising the steps of:
(a) reacting a solution of from about 0.02 to about 3.0 molar of an aromatic or alkyl aromatic compound dissolved in a C₅ to C₁₅ alkane with ceric methanesulfonate to produce a product mixture containing an unreacted aromatic or alkyl aromatic compound, cerous ions and a solid carbonyl-containing product;
(b) separating from said product mixture, a solution of unreacted aromatic or alkyl aromatic compound and recycling said unreacted compound to step (a);
(c) separating the cerous ion from said product mixture;
(d) recovering trace organic substances by liquid - liquid extraction of the electrolyte with an organic solvent;
(e) electrolytically regenerating said cerous ion to form ceric ion and recycling said ceric ion to step (a) for continued processing.

15. A process for producing aromatic quinones, aldehydes or ketones from aromatic or alkyl aromatic compounds, comprising the steps of:
(a) dissolving an aromatic or alkyl aromatic compound in an organic solvent to form a reactant solution, said organic solvent being selected from the group consisting of C₅ to C₁₅ alkanes;
(b) contacting the reactant solution with an aqueous acidic solution of ceric ion to form a product mixture including a liquid aqueous phase, and a liquid organic phase, wherein the concentration of said aromatic or alkyl aromatic compound in said reactant solution is adequate to produce an organic product containing aromatic quinone, aldehyde or ketone in solid form, and wherein the liquid aqueous phase contains cerous ion, and the organic phase contains the organic solvent and unreacted aromatic or alkyl aromatic compounds;
(c) separating the liquid organic phase containing the unreacted aromatic or alkyl aromatic compounds and recycling them to step (b);
(d) separating the liquid aqueous phase including the cerous ion, electrolytically regenerating the cerous ion to ceric ion, and recycling the ceric ion to step (b); and
(e) separating the solid organic product from the product mixture.

16. A process according to claim 1, wherein said organic solvent is substantially free of chlorinated constituents.
